# EUROPEAN PATENT SPECIFICATION

(11) **EP 3 346 937 B1**
(45) Date of publication and mention of the grant of the patent: **17.02.2021**
(21) Application number: 16794407.3
(22) Date of filing: 09.09.2016
(51) Int. Cl.: A61B 18/14, A61B 18/00

(54) **APPARATUS WITH HANDLE ASSEMBLY FOR RADIO FREQUENCY THERAPY**
GERÄT MIT GRIFFANORDNUNG FÜR FUNKFREQUENZTHERAPIE
APPAREIL AVEC UN ENSEMBLE DE POIGNÉES POUR UNE THÉRAPIE PAR RADIOFRÉQUENCE

(30) Priority: 11.09.2015 IT UB20153576
(43) Date of publication of application: 18.07.2018
(73) Proprietor: Novavision Group S.p.A., 20123 Milano (IT)
(72) Inventor: CRAPELLI, Danilo, 20123 Milano (IT)
(74) Representative: Perani & Partners S.p.A.
(86) International application number: PCT/IB2016/055395
(87) International publication number: WO 2017/042737

(56) References cited:
- EP-A1- 2 329 784
- CN-U- 204 092 182
- US-A1- 2009 318 914
- US-A1- 2011 178 584
- US-A1- 2013 245 728

## Description

### Field of the invention

The present invention relates to an apparatus for vaginal radio frequency therapy, with RF energy application handles usable either individually, that is, singularly, or in any predetermined combination thereof.

### Background of the invention

As is known, radio frequency is an electromagnetic radiation range that constitutes the radio spectrum and corresponds to the frequency of electrical signals in alternating current used to produce and/or detect radio waves.

Radio frequency (RF) used in medicine is an advanced technology that is based on the conversion of an electromagnetic wave energy into heat and affects the tissues through the emission of high-frequency radio waves that mimic the thermal effect of lasers.

Radio frequency is similar to optical energy in that it interacts with the tissues, thereby producing a thermal change.

Unlike lasers that produce a warming, thereby selectively hitting a particular chromophore, non-ablative radio frequency generates heat as a result of the tissue resistance to the movement of the electrons subjected to the RF field.

The application of radio frequency energy is able to cause a contraction of the superficial and deep collagen fibers of the body tissues up to the muscle fascia. In the skin, RF radiations cause significant thermal effects at specific depths according to the configuration of the electrodes that transmit them and the type of radio frequency.

The thermal effect degree depends on the conductivity characteristics of the treated tissue, therefore tissues with higher impedance, such as the adipose tissue, generate more heat, resulting in a greater thermal effect.

The European patent EP2329784 by the same Applicant describes a dynamic quadrupole radio frequency technology and a relative safety system for the treatment of blemishes and/or diseases of the human body. The apparatus in which such technology is employed uses a group of electrodes that are configured and managed automatically and dynamically by a control software to circulate the radio frequency current therebetween. The variable configuration of the electrodes as "receivers"/"transmitters" allows creating opposing electric fields that direct the energy in depth in the tissue.

Since the electric fields are generated only within the electrode area in this technology, thermal effects can be achieved in depth and on the desired target by using significantly less RF power than known monopolar systems, thus reducing risks and side effects and improving the treatments and the effectiveness thereof.

The cited European patent software also allows manually setting all the parameters for generating the radio frequency by means of the handles and configuring the electrodes.

In addition, a dedicated safety system implemented within the handles allows the operator to prevent excessive heating of the tissues, thus avoiding unpleasant side effects.

In other words, the system of EP2329784 comprises various parts which, together, contribute to the radio frequency emission monitoring as a function of time and the application position, to prevent a harmful excessive administration of RF energy on the same area of the body for an extended time.

US 2013/0245728 describes a device and a system for remodeling the female genital tissue, the device comprising a treatment tip and one or more energy delivery elements for simultaneous cooling of the vaginal epithelium and transmission of energy for heating the tissues underneath the epithelium. The device may further comprise one or more temperature sensors for measuring the temperature at or below the epithelium; one or more directional sensors mounted on the hand piece or treatment tip, and one or more depth markers to show the depth of penetration of the treatment tip into the vagina.

US 2009/0318914 discloses a device for treating abnormal epithelial tissue of the uterine cervix, such as neoplastic tissue, the device including an operative head supported by a distal portion of the shaft, the head having a distal support surface adapted to conformably engage at least a portion of the cervix, and an energy delivery element on the support surface, the element configured to receive energy from a source and to deliver ablational energy to the tissue in a manner that controls the surface area and depth of ablation. In some embodiments of the device, the energy delivery element is a radiofrequency energy delivery element comprising one or more electrodes.

US 2011/0178584 relates to an apparatus for remodeling a therapeutic zone within tissue underlying a mucosal epithelium of female genital tissue, the apparatus comprising an elongate handle configured to be held within two hands and a treatment tip configured to be removably coupled to the elongate handle, the tip comprising: a shaft comprising a longitudinal axis, an energy delivery element having an epithelium-contacting surface, and an internal cooling chamber configured to internally cool the energy delivery element, wherein the energy delivery element comprises a thermally-conductive surface that is adapted to allow cooling of the epithelium while transmitting RF energy to heat the target tissue

CN 204092182 U relates to a radio frequency therapeutic apparatus used for tightening the vaginal wall with a generator and a a therapeutic handle comprising a handheld component and a therapeutic plug. The therapeutic plug comprises a plurality of radio frequency electrodes are distributed on the surface of the therapeutic plug.

### Summary of the invention

The object of the present invention is to use the RF therapy described in EP2329784 in a new and improved apparatus for the treatment of female vaginal afflictions, in particular vaginal laxity, vulvovaginal atrophy and female stress urinary incontinence.

To this end, it is known that many women experience physical changes after natural childbirth, ranging from sagging breasts to urinary incontinence and can affect the physical well-being of women and their sexuality.

According to doctors, vaginal laxity (also known as vaginal looseness) is the main one among the mentioned physical changes after childbirth, caused by a lengthening of the vaginal opening which occurs naturally during childbirth and causes a marked decrease in physical sensitivity and decreased sexual satisfaction during intercourse.

Currently, Kegel exercises are the most commonly prescribed treatment for vaginal laxity. However, they are scarcely effective for this condition.

Surgical options currently available include significant recovery time and post-procedure pain, as well as a number of health risks and high costs.

A further physical change is vulvovaginal atrophy, directly connected to the lack of production of estrogens by the ovaries, also due to the particular sensitivity of the genital tract and to the decreasing level of such hormones.

Vaginal atrophy symptoms appear approximately 4/5 years after menopause and can affect 50% of women in this stage of their lives. Unlike the other symptoms of menopause, which are generally transient, vaginal atrophy can worsen over the years, with negative effects on sexuality and on the quality of life.

The main changes associated with the decrease in the level of estrogens in menopausal women are due to morphological changes in the vaginal mucosa, such as the thickness of the epithelium and the loss of the vaginal structure; in addition, the blood flow and vaginal secretions are reduced, thereby causing alterations of the vaginal flora.

In terms of sexuality, all these changes have an impact on sexual intercourse, causing dyspareunia and alterations in the female sexual response.

The primary objective of the treatment of vaginal atrophy is indeed to restore the normal physiological conditions of the vagina, so as to reduce the symptoms.

One last substantial physical change is stress urinary incontinence defined as an involuntary loss of urine during normal daily activities, for example because of a cough, a sneeze or exercise.

Urinary incontinence depends on a variety of causes and is connected to the bladder or its sphincter, namely, that thick muscle that controls the flow of urine from the bladder.

Although it is not the age itself to cause incontinence, the normal changes that occur in the urinary and genital system of aging people makes this the most common condition in the elderly.

Any events causing trauma to the pelvic floor - and delivery is one of the most common - can cause stress urinary incontinence.

Currently, the conditions set out above are treated (in addition to surgery and drugs) by promoting and recovering the tissue metabolic activity through a new synthesis activity not only of collagen but also of hyaluronic acid, glycosaminoglycans and proteoglycans. This makes it possible to regain a hydrated mucosa that helps restore the proper tissue tropism, allowing all of the functions of a healthier and younger tissue to be recovered.

The Applicant has found that, by causing the selective heating of the altered vaginal tissues, it is possible to stimulate the production of new collagen and the recovery of the original tone.

Such selective heating of the tissues is currently carried out by laser sources or capacitive radio frequencies which, however, have not proved fully satisfactory, mainly because of their poor suitability to act directly in a targeted manner, that is, in a perfectly controllable manner, on the specific vaginal areas affected by the above diseases.

The task of the present invention therefore is to provide an apparatus for the selective heat treatment of vaginal areas including a set of handles specifically designed to treat, both externally and internally, said vaginal areas by the use of said resistive radio frequency technology.

Within this task, a primary object of the present invention is to provide a set of handles for the indicated use, including a minimum number of handles, for example only three, which may also include contact and contactless thermal sensors for detecting the internal and/or external surface temperature of the vagina, to monitor every moment of the treatment and to verify the efficacy and safety of the treatment itself.

A further object of the present invention is to provide a set of handles which are structurally extremely simple and functional and which can be constructed from commercially available biocompatible and sterilizable materials.

A further object of the present invention is to provide a set of handles, which can be checked and/or operationally managed by the mentioned dynamic quadrupole radio frequency technology with a safety system described in EP2329784.

A further object of the present invention is to provide a set of handles which are easily interchangeable, so as to be able to perform multiple treatments both in the inner cavity and on the outer surface of the vagina, and with the further possibility of treating various diseases and blemishes in this area.

A further object of the present invention is to provide a set of handles, including electrodes adapted to generate electric fields within the vaginal tissues with opposing forces capable of directing the radio frequency energy both vertically and/or horizontally and in depth while reducing the RF energy or power that circulates on the surface, so as to be able to perform effective and comfortable treatments without the use of tissue cooling systems and without harmful side effects.

A further object of the present invention is to provide a set of handles which allow monitoring not only the temperature of the treated vaginal tissue but also the movements of the handles themselves to reduce the risks related to excessive heating of the treated vaginal area.

A further object of the present invention is to provide a set of handles easily mutually connectable, with the configuration and position of the electrodes specifically designed to focus the RF energy on the tissues at any depth desired, without affecting the tissue planes that must not be altered.

A further object of the present invention is to provide a set of handles that do not cause any formation of scar tissue within the vagina and related fibrosis, and which handles at the same time, provide a substantially painless and not annoying treatment of the treated areas.

According to the present invention, the task and the objects mentioned above as well as other objects, which will become more apparent hereinafter, are achieved by an apparatus according to claim 1. Preferred embodiments are defined by the dependent claims.

Aspects, embodiments, examples and implementations of the present disclosure which do not fall within the scope of the appended claims do not form part of the present invention.

According to the present disclosure, a handle is also provided, also referred to as vertical handle, for use in an apparatus for radio frequency therapy of one or more afflictions selected from vaginal laxity, vulvovaginal atrophy, stress urinary incontinence as well as other diseases and/or blemishes of a vaginal area in general, for internal use, wherein said handle is made of a biocompatible and sterilizable material, has a substantially upside-down mushroom-shaped configuration and comprises a substantially cylindrical dome portion with an outer surface and an inner surface substantially flat, a substantially cylindrical shank portion extending substantially perpendicularly from said inner surface, wherein said handle is adapted to generate a uniform heating or a circular selective heating horizontally and is removably supportable by a support handle adapted to connect and disconnect said handle via a coupling system, the handle comprising at least four longitudinal conductive electrodes, mutually spaced apart, the heat in said handle being developed on the length of said longitudinal electrodes and moving circularly under the control of software means. Preferably, the conductive electrodes are of biomedical steel. Preferably, the handle comprises thermal sensors placed between the longitudinal electrodes for detecting the internal temperature of the vagina. In one embodiment, thermal sensors are arranged on one or more longitudinal electrodes. Preferably, the coupling system is safe and quick.

The present disclosure further relates to a handle for use in an apparatus for radio frequency therapy of one or more afflictions selected from vaginal laxity, vulvovaginal atrophy, stress urinary incontinence as well as other diseases and/or blemishes of a vaginal area in general, for external use, said handle being made of a biocompatible and sterilizable material, having a substantially upside-down mushroom-shaped configuration, the handle comprising a substantially cylindrical dome portion with an outer surface and an inner surface substantially flat, a substantially cylindrical shank portion extending substantially perpendicularly from said inner surface, said handle comprising a plurality of conductive electrodes of biomedical steel and being removably supportable by a support handle adapted to connect and disconnect said handle via a safe and quick coupling system. Preferably, the handle for external use is adapted to generate a deep volumetric heating and is usable only on the outer area of the vagina and comprises at least four tip electrodes mutually spaced apart.

### Brief description of the drawings

Further features and advantages of the present invention will become more apparent hereinafter from the following detailed description of some currently preferred embodiments thereof, illustrated by way of non-limiting example in the accompanying drawings, in which:
FIG. 1 is a perspective view of the set of handles which comprises three handles according to the present invention;
FIG. 2 is a perspective view of a common support handle for the three handles in FIG. 1, according to the present invention;
FIG. 2A is a further exploded perspective view showing the main components of the support handle in FIG. 2;
FIG. 3 is a perspective view of a first handle of the set of handles of the present invention;
FIG. 3A is a further exploded perspective view of the main components of the first handle in FIG. 3;
FIG. 4 is a perspective view of the second handle of the set of handles of the present invention;
FIG. 4A is an exploded perspective view of the main components of the second handle in FIG. 4;
FIG. 5 is a perspective view of a third handle for external use of the set of handles of the present invention;
FIG. 5A is a further exploded perspective view of the main components of the handle for external use in FIG. 5; and
FIG. 6 is a block diagram of the radio frequency emitting device associated with the set of handles .

### Detailed description

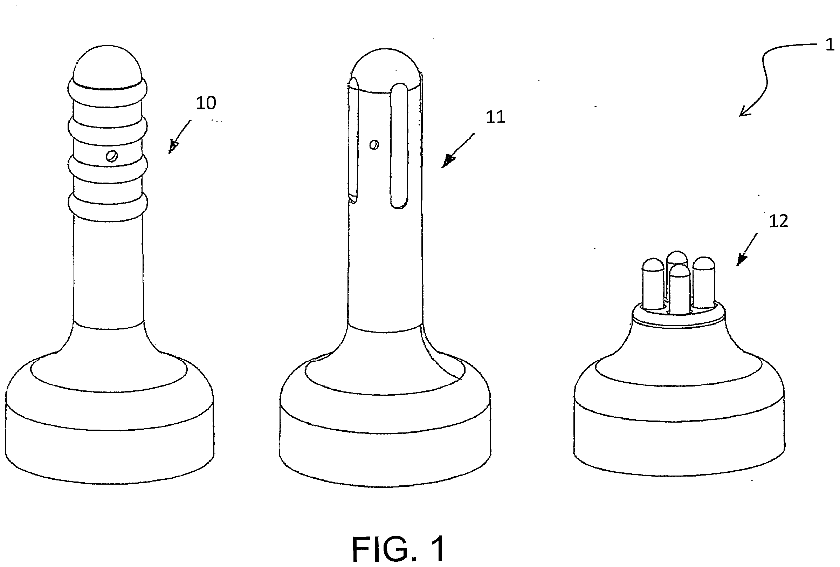

Referring to the above drawings, and in particular to FIG. 1, it shows a perspective view of the set of handles for radio frequency therapy of one or more afflictions selected from vaginal laxity, vaginal atrophy, stress urinary incontinence as well as other diseases and/or blemishes of the vaginal area in general.

The set of handles 1 includes at least three different handles for internal or external use made of biocompatible and sterilizable material, having a substantially upside-down mushroom-shaped configuration, in the orientation shown, with a substantially cylindrical dome portion with an outer surface and an inner surface substantially flat, a substantially cylindrical shank portion extending substantially perpendicularly from the inner surface, the at least three handles comprising a first handle 10 (or horizontal handle, MO), a second handle 11 (or vertical handle, MV) and a third handle for external use 12 (or outer handle, ME), each said handle including a plurality of conductive electrodes of medical steel, and being removably supportable by a common support handle 13 (MS) for the connection and disconnection of each said handle 10, 11, 12 via a safe and quick coupling system.

The handles are sterilizable watertight handles; of a biocompatible material, resistant to high temperatures, for example up to substantially 200 °C; with conductive electrodes advantageously of biomedical AISI 316 or similar steel, mirror-polished to prevent any kind of excoriation to the tissues of the vaginal area; the interior of the handles being resin-coated to prevent penetration of liquids; the contacts between handles 10, 11 and 12 and the support handle 13 being made with golden and cushioned pins; handles 10, 11, 12 being connectable to the respective common support handle 13 via a quick connection with anti-disconnection coupling system.

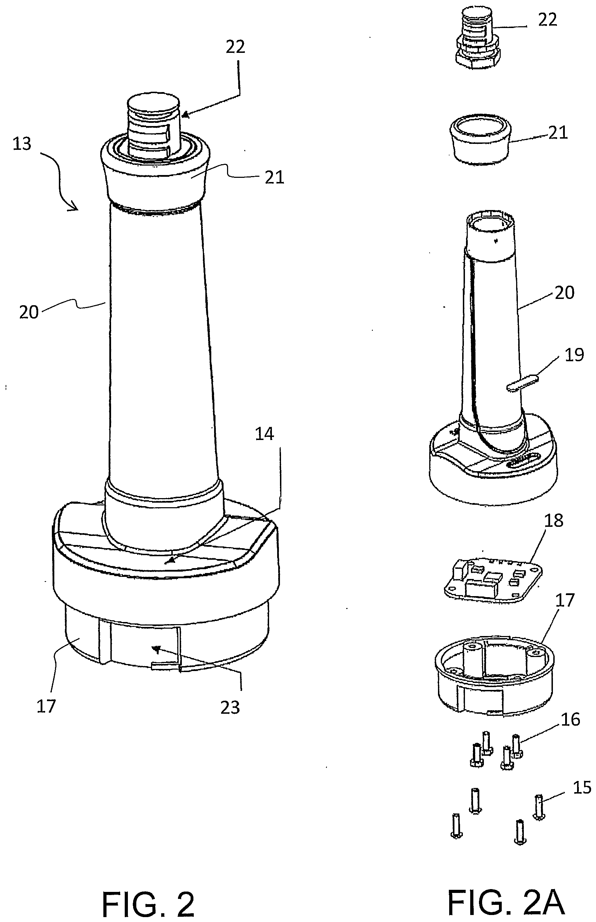

With reference to FIGS. 2 and 2A, they show a perspective view of the support handle 13 with the main components thereof.

Such a handle 13, as said, serves as a common support for the operating or RF energy application handles 10, 11 and 12.

The support handle 13 accommodates control and monitoring electronics 14 of the safety system described in EP2329784.

As can be seen in FIG. 2A, the support handle 13 comprises, from the bottom to the top of the same Figure 2A, a plurality of locking screws 15; a corresponding plurality of connecting screws 16; a lower interface assembly 17; an electronic card 18 which can be accommodated in the interface itself, a handle handling body 20 with a protruding tab 19; an upper connecting ring 21 and finally a terminal top connector assembly 22 for connection to the dynamic quadrupole RF generator. FIG. 2 shows a coupling system 23.

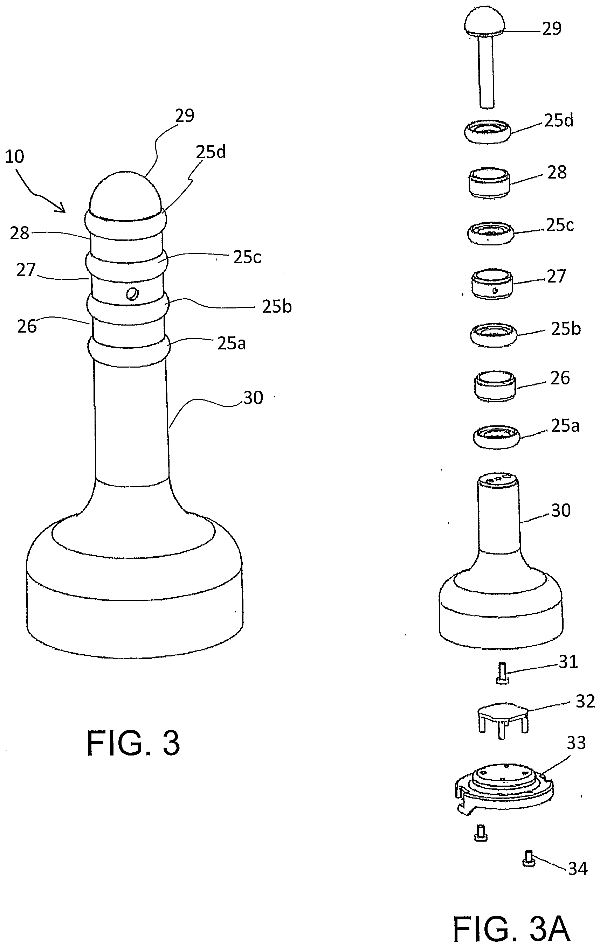

With reference to FIG. 3, it shows a perspective view of the first (horizontal) handle 10 .

Such a first handle 10 is preferably provided with four or more circular electrodes 25a-25d, mutually spaced apart and able to generate a uniform heating or a circular selective heating vertically.

According to the invention, heat is developed starting from the bottom between the first two electrodes 25a and 25b to continue, according to a time set by the apparatus control software, between the second and the third electrode (25b and 25c), between the third and the fourth electrode (25c and 25d), and then back between the first and second electrode 25a and 25b.

Advantageously, handle 10 can be provided with thermal sensors positioned between the various electrodes for detecting the internal temperature of the vagina, to check that the treatment is safe and effective and to prevent undesirable excessive localized heating.

FIG. 3A shows an exploded perspective view of the first handle 10 in FIG. 3.

From the bottom to the top of FIG. 3A, such a horizontal handle 10 comprises bottom locking screws 34, a cover plate 33; a connector board 32; a further handle locking screw 31; a substantially mushroom-shaped handle body 30; a first horizontal electrode 25a engageable on the mushroom-shaped shank of the handle body 30; a first insulation ring 26; a second horizontal electrode 25b; a second insulating ring 27 with positioning of a pyrometric sensor; a third horizontal electrode 25c, a third insulating ring 28, a fourth horizontal electrode 25d (and therefore a total of four horizontal electrodes or perpendicular to the axis of the shank of handle 10) and finally a plastic covering-closing terminal 29.

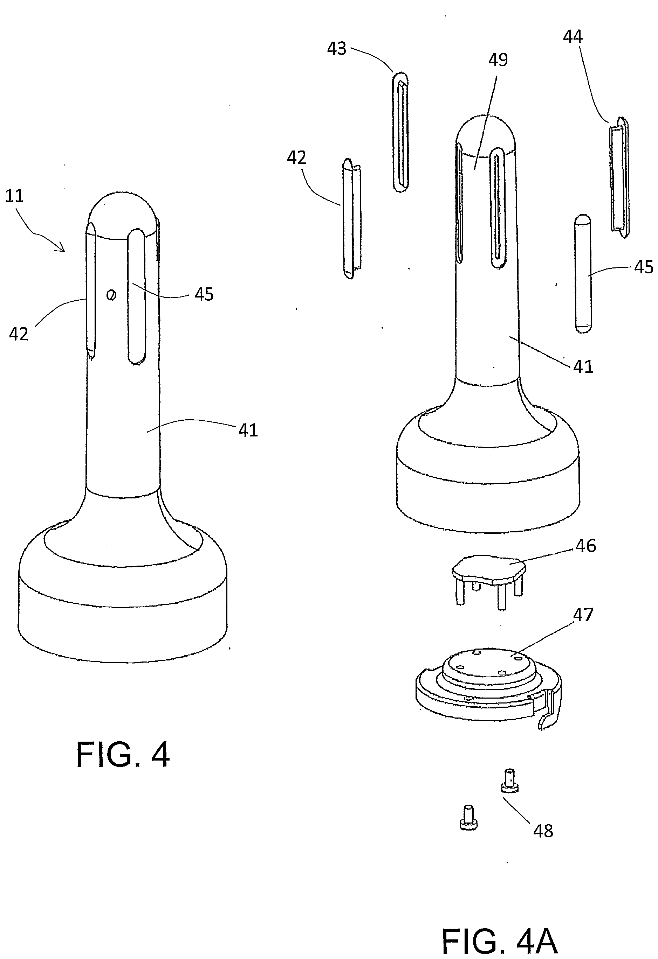

With reference to FIG. 4, it shows a perspective view of the second handle 11 which advantageously comprises at least four longitudinal electrodes 42-45, or parallel to the axis of the handle shank, such as at 90°, angularly mutually spaced apart and advantageously provided for generating a uniform heating or a circular selective heating horizontally. The longitudinal electrodes are arranged vertically, along the axis of the shank of handle 11.

In particular, heat is developed on the length of the vertical electrodes and moves circularly according to a program set by the software.

Advantageously, also the second handle 11 can be provided with thermal sensors placed between the various longitudinal electrodes 42-45 for detecting the internal temperature of the vagina, to check that the treatment is safe and effective and prevent undesirable excessive localized overheating.

FIG. 4A shows an exploded perspective view of the vertical handle 11 in FIG. 4. From the bottom to the top of FIG. 4A, such a vertical handle comprises bottom locking screws 48, a cover plate 47; a connector board 46 which can be removably engaged in the same plate, a substantially mushroom-shaped handle body 41 provided with four circumferentially spaced vertical electrodes 42, 43, 44, 45, as well as a pyrometric temperature sensor. In FIG. 4A, the pyrometric sensor position is indicated with reference numeral 49.

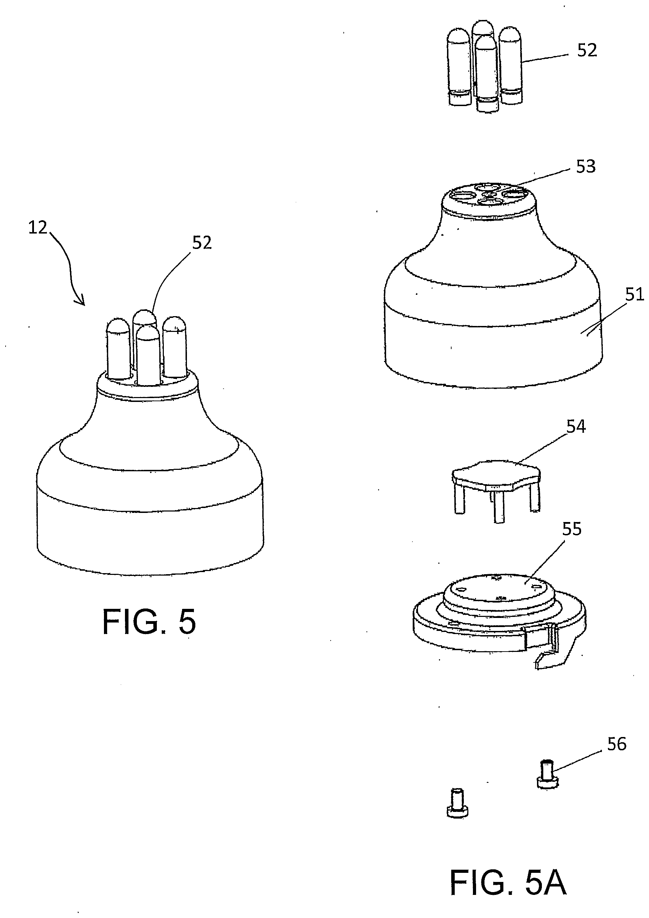

With reference to FIG. 5, it shows a perspective view of the third handle, for external use, which third handle is provided with at least four end tip electrodes, indicated as a whole with reference numeral 52, mutually spaced apart, extending from the top face of the external handle itself and able to generate a deep volumetric heating.

Such an external handle 12 shall be in particular used only on the outer area of the vagina.

The third handle 12 can also advantageously be provided with thermal sensors positioned between the various electrodes for detecting the external temperature of the vagina, to check that the treatment is safe and effective and prevent undesired excessive localized overheating.

With reference to FIG. 5A, it shows an exploded perspective view of the external handle 12 in FIG. 5.

Starting from the bottom of FIG. 5A, the third handle 12 comprises bottom locking screws 56, a cover plate 55, a connector board 54 removably engageable in the cover plate 55, an actual handle body 51, substantially mushroom-shaped and with shortened shank, with top temperature sensors and four tip electrodes 52. Reference numeral 53 indicates the position of the temperature sensor.

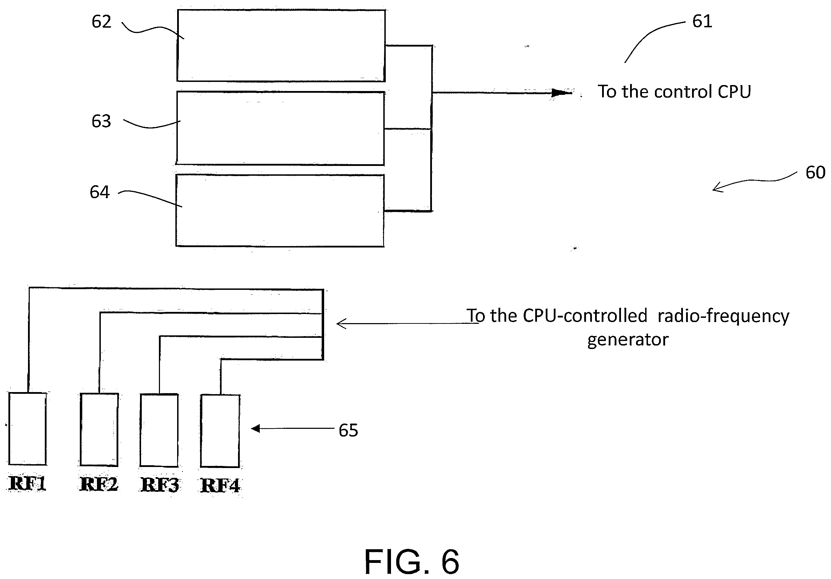

Finally, with reference to FIG. 6, it shows a possible block diagram of a radio frequency emitting device that drives the handles described above. The radio frequency emitting device 60 for the transmission of radio frequency energy to a human body through at least one of the three handles described comprises, operatively functionally interconnected: control means (CPU in the figure) having respective inputs, temperature sensor means 62 and/or motion sensor means 63 and memory means 64 operatively connected to the respective inputs of the control means 61. The control means 61 comprise an output that is operationally connected to and controls, as a function of the memory sensor means, respective contact means 65 of radio frequency generation means to transmit radio frequency energy to at least one vaginal area through each of the described at least three handles, in particular for the radio frequency therapy of one or more vaginal afflictions selected from laxity, vulvovaginal atrophy, stress urinary incontinence, as well as other diseases and/or blemishes of the vaginal area in general.

In one embodiment, the radio frequency generation means is a radio frequency generator controlled by CPU 61. Elements RF1, RF2, RF3 and RF4 in FIG. 6 indicate four biocompatible contacts for the transmission of radio frequency energy to the human body.

Advantageously, in the radio frequency energy emitting device, the temperature sensor means are pyrometric sensor means and/or the like, and the motion sensor means preferably are electronic and/or mechanical motion sensor means adapted to detect the movement of each operating handle, the memory means being adapted to store and save the operating data of each handle.

In one embodiment, the sensor means 62 is a pyrometric or other type of sensor for detecting the temperature of the area to be treated; the motion sensor means 63 is a motion sensor and/or a similar mechanic/electronic circuit that detects the movement of the handle, and the memory means 64 is a memory for saving data of the handle.

While the set of handles and the relevant apparatus to which they are associated and by which they are operatively controlled have been described with reference to a preferred embodiment thereof, they may be subject to numerous modifications and variants, all falling within the scope of the invention as defined in the accompanying claims.

## Claims

1. An apparatus for radio frequency therapy of one or more afflictions selected from vaginal laxity, vulvovaginal atrophy, stress urinary incontinence and other diseases and/or blemishes of the vaginal area in general, comprising:
- a dynamic quadrupole RF generator (60),
- a support handle (13) comprising connection means to the dynamic quadrupole RF generator (60) and a coupling system, and
- a set of RF energy application handles comprising different RF energy application handles (10, 11, 12) for internal and/or external use, said RF energy application handles being made of a biocompatible and sterilizable material, having a substantially mushroom-shaped configuration with a substantially cylindrical dome portion with an outer surface and an inner surface substantially flat, a substantially cylindrical shank portion extending substantially perpendicularly from said inner surface,
wherein said RF energy application handles comprise a first RF energy application handle (10), a second RF energy application handle (11) and a third RF energy application handle (12), each said RF energy application handle comprising a plurality of conductive electrodes of biomedical steel, each said RF energy application handle being removably supportable by said common support handle (13) adapted to connect and disconnect each said first, second and third RF energy application handles via said coupling system,
wherein said connection means are configured for connecting the dynamic quadrupole generator to each of said first, second and third RF energy application handles (10, 11, 12) that can be supported by said support handle (13),
wherein said support handle (13) comprises a safety system having control and mounting means for controlling each of said first, second and third RF energy application handles (10, 11, 12),
wherein the first RF energy application handle (10) comprises at least four circular conductive electrodes (25a-25d) of biomedical steel, mutually spaced apart,
wherein software means are configured to set an operating time, for the first RF energy application handle (10) to develop heat in said first RF energy application handle (10) from the bottom between the first two electrodes (25a, 25b) to continue, on the basis of said operating time set by the software means, between the second and the third electrode (25b, 25c), between the third and the fourth electrode (25c, 25d) and then back between the first and the second electrode (25a, 25b), for said conductive electrodes (25a-25d) to generate a vertically circular selective heating.

2. The apparatus according to claim 1, wherein said first RF energy application handle (10) comprises thermal sensors placed between the various circular electrodes (25a-25d) to detect the internal temperature of the vagina, to check that the treatment is safe and effective and prevent undesired excessive localized overheating.

3. The apparatus according to claim 1, wherein said second RF energy application handle (11) comprises at least four longitudinal electrodes (42-45) mutually spaced apart, the software means being configured to control the second RF energy application handle (11) to generate a uniform heating or a horizontally selective circular heating for the heat in said second RF energy application handle to be developed on the length of said longitudinal electrodes and circularly moving.

4. The apparatus according to claim 3, wherein said second RF energy application handle (11) comprises thermal sensors placed between said longitudinal electrodes (42-45) to detect the internal temperature of the vagina, check that the treatment is safe and effective and prevent undesired excessive localized overheating.

5. The apparatus according to claim 1, wherein the third RF energy application handle (12) is for external use and provided with at least four end tip electrodes (52), mutually spaced apart, extending from the top face of said external RF energy application handle and able to generate a deep volumetric heating.

## Patentansprüche

1. Gerät zur Radiofrequenztherapie für eine oder mehrere Erkrankungen, ausgewählt aus Vaginalschlaffheit, vulvovaginaler Atrophie, Belastungsharninkontinenz und anderen Erkrankungen und/oder, im Allgemeinen, Unreinheiten im Vaginalbereich, umfassend:
- einen dynamischen Quadrupol-RF-Generator (60),
- eine Traghandhabe (13) umfassend Verbindungsmittel zum dynamischen Quadrupol-RF-Generator (60) und ein Kupplungssystem, und
- ein Satz von Handhaben zur Applikation von RF-Energie umfassend verschiedene Handhaben zur Applikation von RF-Energie (10, 11, 12) für den internen und/oder externen Gebrauch, wobei die Handhaben zur Applikation von RF-Energie aus einem biokompatiblen und sterilisierbaren Material hergestellt sind; eine im Wesentlichen pilzförmige Konfiguration aufweisen mit einem im Wesentlichen zylindrischen Kuppelabschnitt mit einer Außenfläche und einer im Wesentlichen flachen Innenfläche; einen im Wesentlichen zylindrischen Schaftabschnitt, der sich im Wesentlichen senkrecht zur Innenfläche erstreckt,
wobei die Handhaben zur Applikation von RF-Energie eine erste Handhabe zur Applikation von RF-Energie (10), eine zweite Handhabe zur Applikation von RF-Energie (11) und eine dritte Handhabe zur Applikation von RF-Energie (12) umfassen; jede Handhabe zur Applikation von RF-Energie eine Vielzahl von leitenden Elektroden aus biomedizinischem Stahl umfasst; jede Handhabe zur Applikation von RF-Energie von der gemeinsamen Traghandhabe (13) lösbar aufnehmbar ist, die zum Verbinden und Trennen jeder ersten, zweiten und dritten Handhabe zur Applikation von RF-Energie über das Kupplungssystem ausgelegt ist,
wobei die Verbindungsmittel konfiguriert sind, um den dynamischen Quadrupol-Generator mit jeder ersten, zweiten und dritten Handhabe zur Applikation von RF-Energie (10, 11, 12) zu verbinden, die von der Traghandhabe (13) getragen werden können,
wobei die Traghandhabe (13) ein Sicherheitssystem umfasst, aufweisend Steuer- und Befestigungsmittel zur Steuerung jeder der ersten, zweiten und dritten Handhaben zur Applikation von RF-Energie (10, 11, 12),
wobei die erste Handhabe zur Applikation von RF-Energie (10) mindestens vier kreisförmige leitende Elektroden (25a-25d) aus biomedizinischem Stahl umfasst, die voneinander beabstandet sind,
wobei Softwaremittel konfiguriert sind, um eine Betriebszeit für die erste Handhabe zur Applikation von RF-Energie (10) zu setzen, um Wärme in der ersten Handhabe zur Applikation von RF-Energie (10), von unten zwischen den ersten beiden Elektroden (25a, 25b) zu entwickeln und weiter, auf der Grundlage der von den Softwaremitteln gesetzten Betriebszeit, zwischen der zweiten und der dritten Elektrode (25b, 25c), zwischen der dritten und der vierten Elektrode (25c, 25d) und dann zurück zwischen der ersten und zweiten Elektrode (25a, 25b), eine kreisförmige selektive vertikale Erwärmung für die leitenden Elektroden (25a-25d) zu erzeugen.

2. Gerät nach Anspruch 1, wobei die erste Handhabe zur Applikation von RF-Energie (10) thermische Sensoren umfasst, die zwischen den verschiedenen kreisförmigen Elektroden (25a-25d) angeordnet sind, um die Innentemperatur der Vagina zu erfassen, um zu überprüfen, ob die Behandlung sicher und wirksam ist, und um unerwünschte übermäßige lokale Überhitzung zu verhindern.

3. Gerät nach Anspruch 1, wobei die zweite Handhabe zur Applikation von RF-Energie (11) mindestens vier Längselektroden (42-45) umfasst, die voneinander beabstandet sind; die Softwaremittel zur Steuerung der zweiten Handhabe zur Applikation von RF-Energie (11) konfiguriert sind, um eine gleichmäßige Erwärmung oder eine kreisförmige selektive horizontale Erwärmung für die Wärme in der zweiten Handhabe zur Applikation von RF-Energie zu erzeugen, die sich entlang der Länge der Längselektroden und kreisförmig bewegend entwickelt.

4. Gerät nach Anspruch 3, wobei die zweite Handhabe zur Applikation von RF-Energie (11) thermische Sensoren umfasst, die zwischen den Längselektroden (42-45) angeordnet sind, um die Innentemperatur der Vagina zu erfassen, um zu überprüfen, ob die Behandlung sicher und wirksam ist, und um unerwünschte übermäßige lokale Überhitzung zu verhindern.

5. Gerät nach Anspruch 1, wobei die dritte Handhabe zur Applikation von RF-Energie (12) für den externen Gebrauch bestimmt ist und mit mindestens vier voneinander beabstandeten Endspitzenelektroden (52) versehen ist, die sich von der Oberseite der äußeren Handhabe zur Applikation von RF-Energie erstrecken und eine tiefe volumetrische Erwärmung erzeugen können.

## Revendications

1. Appareil pour thérapie par radiofréquence d'une ou plusieurs indispositions choisies parmi la laxité vaginale, l'atrophie vulvo-vaginale, l'incontinence urinaire de stress et autres maladies et/ou imperfections de la zone vaginale en général, comprenant :
- un générateur RF quadripôle dynamique (60),
- une poignée de support (13) comprenant des moyens de connexion au générateur RF quadripôle dynamique (60) et un système de couplage, et
- un ensemble de poignées d'application d'énergie RF comprenant différentes poignées d'application d'énergie RF (10, 11, 12) pour une utilisation interne et/ou externe, lesdites poignées d'application d'énergie RF étant réalisées en un matériau biocompatible et stérilisable, ayant une configuration sensiblement en forme de champignon avec une portion de dôme sensiblement cylindrique avec une surface externe et une surface interne sensiblement plates, une portion de tige sensiblement cylindrique s'étendant sensiblement perpendiculairement de ladite surface interne,
dans lequel lesdites poignées d'application d'énergie RF comprennent une première poignée d'application d'énergie RF (10), une deuxième poignée d'application d'énergie RF (11) et une troisième poignée d'application d'énergie RF (12), chaque dite poignée d'application d'énergie RF comprenant une pluralité d'électrodes conductrices en acier biomédical, chaque dite poignée d'application d'énergie RF pouvant être supportée de manière amovible par ladite poignée de support (13) commune adaptée pour connecter et déconnecter chaque première, deuxième et troisième poignée d'application d'énergie RF par l'intermédiaire dudit système de couplage,
dans lequel lesdits moyens de connexion sont configurés pour connecter le générateur quadripôle dynamique à chacune des première, deuxième et troisième poignées d'application d'énergie RF (10, 11, 12) qui peuvent être supportées par ladite poignée de support (13),
dans lequel ladite poignée de support (13) comprend un système de sécurité ayant des moyens de commande et de montage pour commander chacune des première, deuxième et troisième poignées d'application d'énergie RF (10, 11, 12),
dans lequel la première poignée d'application d'énergie RF (10) comprend au moins quatre électrodes conductrices circulaires (25a-25d) en acier biomédical, mutuellement espacées,
dans lequel des moyens logiciels sont configurés pour fixer un temps de fonctionnement, pour que la première poignée d'application d'énergie RF (10) développe de la chaleur dans ladite première poignée d'application d'énergie RF (10) depuis le bas entre les deux premières électrodes (25a, 25b) pour continuer, sur la base dudit temps fixé par les moyens logiciels, entre la deuxième et la troisième électrode (25b, 25c), entre la troisième et la quatrième électrode (25c, 25d) et puis de nouveau entre la première et la deuxième électrode (25a, 25b), pour que lesdites électrodes conductrices (25a-25d) génèrent un chauffage sélectif circulaire vertical.

2. Appareil selon la revendication 1, dans lequel ladite première poignée d'application d'énergie RF (10) comprend des capteurs thermiques positionnés entre les différentes électrodes circulaires (25a-25d) pour détecter la température interne du vagin, pour vérifier que le traitement est sûr et efficace et prévenir une surchauffe excessive localisée non désirée.

3. Appareil selon la revendication 1, dans lequel ladite deuxième poignée d'application d'énergie RF (11) comprend au moins quatre électrodes longitudinales (42-45) mutuellement espacées, les moyens logiciels étant configurés pour contrôler la deuxième poignée d'application d'énergie RF (11) pour générer un réchauffement uniforme ou un réchauffement circulaire sélectif pour que la chaleur dans ladite deuxième poignée d'application d'énergie RF soit développée sur la longueur desdites électrodes longitudinales et se déplace de manière circulaire.

4. Appareil selon la revendication 3, dans lequel ladite deuxième poignée d'application d'énergie RF (11) comprend des capteurs thermiques positionnés entre lesdites électrodes longitudinales (42-45) pour détecter la température interne du vagin, vérifier que le traitement est sûr et efficace et prévenir une surchauffe excessive localisée non désirée.

5. Appareil selon la revendication 1, dans lequel la troisième poignée d'application d'énergie RF (12) est pour une utilisation externe et est fournie avec au moins quatre électrodes de pointe d'extrémité (52), mutuellement espacées, s'étendant de la face supérieure de ladite poignée d'application d'énergie RF externe et capables de générer un réchauffement volumétrique profond.
